# EUROPEAN PATENT APPLICATION

(11) **EP 4 717 220 A1**
(43) Date of publication of application: **01.04.2026**
(21) Application number: 25191577.3
(22) Date of filing: 24.07.2025
(51) Int. Cl.: A61B 34/10, A61B 34/20, A61B 90/40, A61B 90/00, G02B 27/20

(54) **PROJECTION OF A PLANNED PATIENT SPECIFIC IMPLANT MODEL**

(30) Priority: 30.09.2024 WO PCT/EP2024/077480
(71) Applicant: Brainlab SE, 81829 München (DE)
(72) Inventor: WEISER, Manfred, 81829 München (DE); UHDE, Jörg, 81829 München (DE)
(74) Representative: Maiwald GmbH

(57) **Abstract**

A system and method for generating a projection of a planned patient specific implant model 101 on a sterile projection plate 102 in a sterile field is presented. The method comprises the steps of detecting the sterile projection plate 102 located in the sterile field with a detection device 103 (step S1) and calculating, based on a result of the detection of the sterile projection plate carried out in step S1, projection parameters for the projection device (step S4). Further, the planned patient specific implant model is provided to a projection device 104 (step S2). Further the step of projecting the planned patient specific implant model 101 with the projection device 104 onto the sterile projection plate (step S3) is comprised, wherein said calculated projection parameters in step S3 when projecting the planned patient specific implant model with the projection device onto the sterile projection plate (step S5). The method shown in Figure 1 can be carried out by a system as shown in Figure 1, but also other systems may be used.

## Description

### FIELD OF THE INVENTION

The present invention relates to a method of generating a projection of a planned patient specific implant model on a sterile projection plate in a sterile field and relates to a system for generating a projection of a planned patient specific implant model on a sterile projection plate in a sterile field.

### TECHNICAL BACKGROUND

Implanting processes entail several complexities. For example, many spinal surgeries that are performed to reduce pain or restore biomechanical alignment of the spine rely on intervertebral fusion. To induce such a fusion and to control the alignment correction, screw-rod constructs (hereinafter referred to as rods) are used. The screws function as the anchors within each vertebral level while the rods interconnect the screws, preventing motion and dictating the local alignment in this area. This, together with further spinal disc removal, placement of intervertebral implants, including bone graft material as well as facet joint preparation or additional plate placements, starts the fusion process.

The rods hereby also induces the total biomechanical alignment after the fusion. The process of fitting the rod to a shape that shall induce a certain profile to the fusion area, in combination with additionally fitting the rod through individually placed screws, is a complex task. Similar reasons hold true for the complexity of other implant processes.

Moreover, today's spinal surgery planning software is sometimes used to design a rod shape that shall induce a favorable biomechanical alignment. These shapes are then either sent digitally to 3D printing companies (e.g. "Medicrea") or they are translated into instructions for a step-by-step bending assistance ("Bendini" Nuvasive). The creation of a sterile template for manual rod bending is currently a cumbersome process, manually performed by e.g. printing shapes at scale and manually placing the printouts in sterile bags or boxes, see the sterile encasing device described in the patent application WO23021204 A1. However, the inventors of the present invention identified that static print-out on paper doesn't adapt to changes, requires a sterile box, which is an expensive disposable, and also the user experience is bad, because the process of printing is still needed. Moreover, printers need to be individually calibrated to keep the scale, which is cumbersome and error prone. The inventors also found that since paper itself is flexible, it is therefore not good for being placed flat on a patient, i.e., the usability is to be improved.

These insights of the inventors of the present invention made them identify the need for a method and a system which reduces such complexities in general for implanting processes.

Aspects of the present invention, examples and exemplary steps and their embodiments are disclosed in the following. Different exemplary features of the invention can be combined in accordance with the invention wherever technically expedient and feasible.

### EXEMPLARY SHORT DESCRIPTION OF THE INVENTION

In the following, a short description of the specific features of the present invention is given which shall not be understood to limit the invention only to the features or a combination of the features described in this section.

The disclosed method of generating a projection of a planned patient specific implant model on a sterile projection plate in a sterile field encompasses the detection of a sterile projection plate located in the sterile field with/by a detection device, i.e., step S1. In step S2, the planned patient specific implant model is provided to a projection device, and in step S3, projecting the planned patient specific implant model with said projection device onto the sterile projection plate is carried out. It must be noted that the order of the method steps may vary. For example, the provision of the implant model, i.e., step S2, may be carried out before, during or after step S1 is carried out. Other variations are also included in the method presented herein.

It must be noted that for all method embodiments of the present invention presented herein the system of the present invention has a corresponding configuration such that the system and its components, i.e., the detection device and the projection device, as will be detailed hereinafter can carry out the presented method embodiment. This will be detailed in the following.

The method and system of the present invention, which generate said one or more projections of one or more planned patient specific implant models with the projection device onto the sterile projection plate, may be seen as the provision of a digital template or digital stencil of the real implant on the surface of the sterile plate in the sterile field. The provision is achieved by projecting a planned implant model, which is patient specific. Thus, in a pre-process before the projection into the sterile field, the implant model is typically planned by medical personnel and the resulting patient specific implant is described by and stored in a digital file. This planning of the model of the implant is e.g. done by utilizing a digital spinal-alignment planning application, that loads digitized patient data (e.g. CT and / or X-ray data), scales, classifies and detects spinal features (e.g. "vertebrae detection", "automatic labelling", "automatic measurements") and allows in a next step the manual or automated realignment of the spinal anatomy. Such realigned anatomy (following e.g. anatomical guidelines or being optimized by atlas or machine learning output) then defines one or more appropriate rod-shapes, i.e. individual digital models of the implant, which will be used later on for a particular, individual patient to implement the planned spinal-alignment. Files containing and/or defining the planned patient specific implant model or models may be stored in the system of the present invention detailed hereinafter, but may also be stored somewhat remote on a server in a digital cloud and may be accessible by the system for projecting the planned patient specific implant model or models onto the sterile projection plate in the sterile field.

The sterile field is understood by the skilled reader as a section, zone or space of an operating room of a hospital or a similar medical care centre. The sterile field is understood as the space around the patient positioned on the surgical table that is kept in sterile condition during surgery, including the surgeon(s), sterile nurse(s), and sterile instruments on a sterile table.

The planned patient specific implant model, i.e., the model, in an embodiment may use finite element modelling (FEM), but the skilled person will appreciate other techniques as alternatives for preparing or providing the planned patient specific implant model.

The "planned patient specific implant model" as used herein may be a model of a real implant, preferably of a bendable real implant, i.e., which can be bent by a user during the implanting process. This may include bending the real implant during actually implanting the implant, and/or may include the preparation of the implant, more or less immediately, before insertion.

The system for generating a projection of a planned patient specific implant model onto a sterile projection plate in a sterile field of the present invention may use a detection device, like e.g. a video camera and a projection device, like e.g. a laser. As is clear from the present description and is understood by the skilled reader, such devices must be accordingly configured to be able to carry out the method as presented hereinbefore. In particular embodiments of the system, software, i.e., a computer program or a computer program element is comprised for carrying out the projection of the planned patient specific implant model onto the sterile projection plate in the sterile field. The sterile or sterilizable projection plate used in the context of the present invention is preferably flat, but also other shapes may be used, as will be explained hereinafter.

In general, with the projection provided by the present invention the user may, for example during spinal surgeries, use this digital template or digital stencil of the real implant projected onto the surface of the sterile plate in the sterile field as a guidance or support information during an implanting process. As is understood by the skilled reader, the visual appearance of the projection, i.e. the visual appearance of the planned patient specific implant model on the sterile plate, provides for the user a reference object in the sterile field during e.g. the bending of real implants like for example screw-rod constructs. As will be appreciated by the skilled reader, the visual appearance of the planned patient specific implant model on the sterile plate can of course also be advantageously used in other scenarios.

With the present invention the cumbersome process for creating a sterile template for manual implant shaping/bending, which is currently done in the prior art, can be avoided. Moreover, also the need to print implant shapes at 1:1 scale and manually placing the printouts in sterile bags or boxes is not necessary anymore. As was mentioned hereinbefore, the static print-out on paper used in the prior art doesn't adapt to changes and requires an expensive sterile box. In contrast, with the present invention, advantageously no such sterility measures need to be present, since the digital projection onto the sterile plate per se does not negatively affect the required sterility. Only the sterile plate must be provided which can be an off-the-shelf, standardized product. Moreover, as will be explained in the context of embodiments hereinafter, the digital character of the projection used in the present invention facilitates a fast and easy way of adapting the projection in case the projection surface, i.e. the surface of the sterile plate, is moving. According to embodiments, such movements of the sterile plate can be detected by the method/system, preferably by the detection device, such that these movements are compensated for by accordingly adapting the projection on the moving sterile plate. This allows for real-time scaling of the projected implant model based on the detected movement of the sterile plate and/or allows for real-time calibration. Note that it is not meant that the model is permanently rescaled. Rather, preferably, the projection shall be such that the display is always scaled 1:1 to the model, i.e. size is conserved. Regarding the "calibration", a "live calibration" is preferably meant, where the system and/or the method can compensate for a moving plate by detecting said movement and by accordingly adapting the projection, thereby preserving the 1:1 scaling. As is clear to the skilled reader, the solution presented herein, i.e., the projection of the implant model into the sterile field, can thus beneficially be used for supporting a user in improving implanting processes.

As will become apparent from the following disclosure, in particular embodiments of the present invention the method and system of the present invention is capable of projecting the planned patient specific implant model with the projection device onto the sterile projection plate at scale.

### GENERAL DESCRIPTION OF THE INVENTION

In this section, a description of the general features of the present invention is given for example by referring to possible embodiments of the invention.

According to a first aspect of the present invention, a method of generating a projection of a planned patient specific implant model on/onto a sterile projection plate in a sterile field is presented. The method comprises the steps of detecting a sterile projection plate located in the sterile field with a detection device (step S1), providing the planned patient specific implant model to a projection device (step S2), and projecting the planned patient specific implant model with the projection device onto the sterile projection plate (step S3).

Thus, with the presented method it is suggested to make a template of the implant, i.e. a planned patient specific implant model, available in the sterile field by using a combination of a detection device, like e.g. a video camera, and a projection device, like e.g. a laser projection device. It will become apparent form the present disclosure, that the method may also use other detection devices and other projection devices. As is clear to the skilled reader the presented method is device implemented, i.e., carried out by the system as disclosed herein. It may preferably be seen as a computer controlled or computer implemented method.

The inventors found that by carrying out a detection of the sterile plate, the provision of an implant model and the projection of the model onto the plate in the sterile field many disadvantages of the prior art mentioned hereinbefore are improved or at least partly overcome.

In step S1, the sterile projection plate is detected with the detection device. For example, the detection device may "know" the sterile plate due to information/parameters about the sterile plate, which is accessible by the system. Such information or parameters may be stored in the system, e.g., in the detection device, but this may also be stored remotely, for example on a server. Based on said information/parameters the system, preferably the detection device (e.g. a camera or video camera), is configured to identify/detect the sterile plate. For example, the information/parameters define the size and/or shape of the sterile plate and hence the detection device, by using e.g. well-known object detection algorithms, can detect or identify the sterile plate in e.g. pictures of the camera or video camera. Based on this detection or identification of the sterile plate, the method and system of the present invention can be configured to calculate how to project the implant model onto the sterile plate.

Due to the fact that a sterile plate is used, the presence of the sterile plate is not negatively affecting the sterility of the sterile field.

Moreover, as was mentioned before, the presented method and the corresponding system can be used for several different types of implants. In the following, several advantages and aspects of the method and system will be described by way of examples like e.g. the rods used in spinal surgeries mentioned hereinbefore. This is, however, only one example and shall be understood of not limiting the present invention.

As is clear to the skilled reader, the shape of the implant, e.g. the rod, dictates and herby also induces the total biomechanical alignment after the implanting process. The process of fitting the implant, e.g. the rod, to a shape that shall induce a certain profile to the implanting area, in combination with additionally mechanically and manually shaping, i.e., fitting, the implant through e.g. individually placed screws, is a complex task, and this complexity is beneficially reduced by the provision of the projected planned patient specific implant model onto the sterile projection plate in the sterile field where the user is working. For reducing this complexity, the present invention makes a template, i.e. the projected model, available in the sterile field, which template can easily be used for manually implant bending or shaping. By using a combination of detection device and a projection device to project the implant model, preferably a scaled patient specific implant model on a sterile projection plate in the sterile field. The method and system may also make us of software, computer programs and/or computer program elements for the detection and/or the projection, as will be explained in more detail hereinafter in the context of particular embodiments. In general, the patient specific implant model may be the result of previous planning, e.g. in Spine Planning or the like.

It will also become apparent from the following disclosure that by tracking of the real implant, i.e. by using a marker-based or a marker-less tracking method and devices known in the art, the implant model, i.e. the template, can beneficially be projected in different orientations. This is part of a particular embodiment described in detail hereinafter. Note that one possible benefit is that by detecting the position of the physical rod, but especially its rotation, the system and method of this embodiment could always project the "best matching projection direction" of the 3D-template onto the sterile plane. Another benefit would be to allow for real time surveillance and additional guidance for the process of adapting the physical rod to a fixed projection. The system and method could e.g. issue a warning that the user has rotated the rod along the axis, and request the user to rotate it back, bevor he/she continues bending, or it provide a guiding in the sense of "look to this point here, you need more bending".

With the presented method the following procedure is facilitated, which may be seen as a particular embodiment. The projection plate can be detected with the detection device. If desired, the detection device can be calibrated to the projection device. The planned patient specific implant model, e.g. of a rod, can be provided. Thus, planning data or digitization data may be provided to the system of the present invention, or the system gets access to a data storage soring these data. Further, with the projection device the planned implant model can be projected, preferably in correct size, based on the detection of the projection plate. This enables the user to compare the real implant to the projected template, i.e., the projected planned patient specific implant model. The projection device and the detection device, optionally also a tracking device allowing views in various directions, may be provided in a combined device, i.e. in one housing. Registration of the projection device and the detection device relative to each other via a calibration step is possible and covered by the present disclosure. If desired, scaling and further information can be shown/displayed to the user, preferably on the projection plate. Thus, scaling information may be projected/displayed to the user by the system, e.g. the projection device. In other words, augmented information of one or more implant parameters may be provided by the method/system for the user in this way.

In a particular embodiment, the detection device is configured to detecting the implant shape and/or implant surface and/or implant orientation. This detection can be used to provide updates, measurements, quality control, indicator for deviation, and/or replanning information for the user, as will be described in more detail in the context of particular embodiments hereinafter. This detection can also be used to provide a project workflow to the user by projecting intermediate states of the implant model during a bending process, i.e., providing an update of the implant model according to bending progress. In another particular embodiment, the method and system of the present invention are configured to track the real implant by e.g. video or marker-based to show correct position and orientation of the projected implant model to the user. This will be explained in more detail hereinafter in the context of particular embodiments.

According to an embodiment of the present invention, the method further comprises the step of determining, upon detecting the sterile projection plate located in the sterile field with the detection device carried out in step S1, a relative position and a relative orientation of the sterile projection plate to the detection device.

In this embodiment, it is specified that the relative position and the relative orientation of the plate to the detection device is determined. It is then detailed in the next embodiment that knowing these parameters allows the method and system of this embodiment to properly scale and display an undistorted projection of the planned patient specific implant model on the sterile projection plate.

According to an embodiment of the present invention, the determined relative position and the determined relative orientation of the sterile projection plate to the detection device facilitate a correct scaling and an undistorted projection of the planned patient specific implant model onto the sterile projection plate.

While the user may in principle also use the presented method and/or system with a projection which comprises some degrees of incorrect scaling and/or distortions, with this embodiment an error free, realistic projection can be provided to the user based on the relative position and the relative orientation that was previously determined by the method and/or the system. Based on this disclosure the skilled person might use known techniques for determining the position and orientation of an object relative to another object in one coordination system based on e.g. an image or a video, i.e., based on an image or a video of the sterile plate. For example, based on the known shape of the projection plate and the known optical parameters of the optical system (camera calibration), the pose of the plate with respect to its position and angular orientation can be determined from the observed shape which appears distorted and scaled due to the projective nature of the imaging.

According to an embodiment of the present invention, the method further comprises the step of calculating, based on a result of the detection of the sterile projection plate carried out in step S1, projection parameters for the projection device (step S4), and using said calculated projection parameters in step S3 when projecting the planned patient specific implant model with the projection device onto the sterile projection plate (step S5).

In this embodiment aspects of the process of projecting the model are detailed. It describes how the system and the method of this embodiment can define the used projection parameters based on the detection of the sterile plate. It must be noted that the calculation of step S4 can be carried at varies instances/devices. For example, the calculation of the projection parameters for the projection device can be carried out in the detection device and/or in the projection device. However, the calculation may also be carried out on a remote calculation device like e.g. a remote server to which the system and method of the present invention have access. Via a communication channel the result of the detection of the sterile projection plate carried out in step S1 as well as the calculated projection parameters can be transmitted to and from such a remote calculation device to the system of the present invention. In case the projection parameters are calculated with the projection device, a communication channel between the detection device and the projection device is used to transmit the result of the detection of the sterile projection plate carried out in step S1 to the projection device.

According to an embodiment of the present invention, using said calculated projection parameters ensures a correct scaling and an undistorted projection of the planned patient specific implant model onto the sterile projection plate.

As is clear to the skilled reader from this disclosure, in this embodiment the system of the presented embodiment, e.g. the camera used as the detection device, knows or has available the size of the plate and uses this information for identifying the plate. In other words, the camera or the identification device in general knows the size of the plate and hence can, upon having made an image of the actual plate being positioned in the sterile field, identify or detect the plate based on the combination of this image and the size information stored within the detection device, or elsewhere and the detection device or system is configured to get access and gets access to the stored information. Known image-based object detection algorithm may be used in this context.

According to an embodiment of the present invention, in the detection device and/or in the projection device and/or on/in the sterile plate identification information about the sterile projection plate is stored, the method further comprising the steps,
generating at least one image of the sterile projection plate by the detection device, and
identifying the sterile projection plate by the detection device based on a comparison between the stored identification information and content of the generated at least one image.

The stored identification information may be parameters about the visual representation of the sterile plate, like the color, the size, or information about shape and distribution of additional detection and identification markers such as additional lines, corners, rings, triangles, boxes, circles, crosshairs, checkboard patterns or combinations of such features, that additionally may serve the purpose to improve the robustness of the detection, or may e.g. be a QR code or a UPC barcode that is provided on the surface of the sterile plate and which the detection device can scan with an imaging sensor and/or a camera sensor for identifying the plate. Besides a QR code, which is a type of two-dimensional matrix barcode, also other coding techniques may be used for providing the system of the presented embodiment with the identification information. A QR code features black squares on a white background with fiducial markers, readable by imaging devices like cameras, and processed using Reed-Solomon error correction until the image can be appropriately interpreted. The required data are then extracted from patterns that are present in both the horizontal and the vertical components of the QR image on the sterile plate.

According to an embodiment of the present invention, in the detection device and/or in the projection device and/or elsewhere identification information about the size and the shape and possible additional identification parameters of the sterile projection plate is stored. And the method comprises the step of calculating, based on the generated at least one image and the stored identification information, projection parameters for the projection device for projecting the planned patient specific implant model onto the sterile projection plate.

Due to the identification of the sterile plate based on the stored identification information, this embodiment focuses on the desired correct scaling and/or undistorted projection of the planned patient specific implant model onto the sterile projection plate. This can be achieved when the projection parameters to be used by the projection device are calculated accordingly. For example, the camera may know the size, shape and possible additional identification parameters of the plate and uses this for the identification of the plate and the determination of its spatial orientation with respect to the detection device. This may then be used for calculating proper projection parameters based on which an improved projection can be provided by the projection device. This embodiment takes into account that generally knowing the size would not be sufficient in case the sterile plate is not rectangular. In order to cover any kind of shaped plates, so called dimensioned form-information, i.e., size and shape information, should be provided, which is realized in the presented embodiment.

Additionally, further identification parameters of the sterile projection plate can be provided or stored on the surface of the sterile plate that can be detected by the detection device in order increase robustness of the detection process, i.e. the detection result. However, in case the sterile plate has a rectangular shape, the following embodiment may advantageously be used.

According to an embodiment of the present invention, in the detection device and/or in the projection device and/or elsewhere size information about a size and the shape of the sterile projection plate is stored. And the method comprises the step of calculating, based on the generated at least one image and the stored size information, projection parameters for the projection device for projecting the planned patient specific implant model onto the sterile projection plate.

As mentioned before, this embodiment may be particularly helpful in case the sterile plate is of rectangular shape and hence no additional information about the size is needed.

According to an embodiment of the present invention, the detection device is a video camera, and/or the projection device is a laser projection device.

The camera may be a photo camera, a video camera, a surface camera, or a sensor capable of identifying objects. The projection device may user laser technologies of various technology, but may also use non-laser projection technologies, like e.g., LED or OLED components to carry out the desired projection of the implant model.

According to an embodiment of the present invention, the method further comprises the step of calibrating the detection device to the projection device with respect to their spatial relative position and/or their relative angular orientation.

In this embodiment it is described that the detection device and projection device are calibrated relative to each other. Such a calibration can be done once in case they are combined in one housing, or generally speaking in case they have a fixed position relative to each other within the system of the present invention. **In** other embodiments, the calibration for these two devices is carried out frequently in case these two devices can be moved relative to each other and hence do not have a fixed position and/or a fixed orientation to each other.

According to an embodiment of the present invention, the method further comprises the steps
receiving tracking data indicative of a position and/or orientation of a real implant or of a surgical instrument, and
projecting the planned patient specific implant model in different orientations onto the sterile projection plate with the projection device based on the received tracking data.

By tracking of the real implant, the projected patient specific implant model can be projected in orientations, related to a certain actual rotation or position of the real implant. In other words, the tracking facilitates a matching orientation. Preferably, the projection of the patient specific implant model can be adapted in real time based on or follow the current orientation and/or position of the tracked real implant. This may be used as a real-time adaptation of projection of the patient specific implant model based on the currently detected orientation and/or position of the real implant. Tracking the position and/or rotation of an implant, e.g. the rod model can be advantageous as e.g. a rod could be planned to be bent in different planes. The projection shows only one plane at a time. Observing the rotation of the rod allows to project the template for bending also in alternative planes.

According to an embodiment of the present invention, the tracking data are video-tracking data and/or marker-based tracking data.

In general, the tracking can be carried out by e.g. the detection device, but it may also be carried out by a separate tracking device. The skilled reader will appreciate that various different known tracking technologies like optical tracking, non-optical tracking, marker-based tracking and marker-less tracking can be used.

According to an embodiment of the present invention, the method comprises the steps
detecting a shape, a surface and/or an orientation of a real implant, preferably by using an object detection algorithm,
comparing the detected shape, surface and/or orientation of the real implant with a plan for implanting the planned patient specific implant model, and
detecting at least one deviation from said plan based on a result of said comparing.

This embodiment provides support for the user for adapting the real implant exactly to a planned implant which is derived from an implantation plan. With this embodiment the method/system provides a control of the implant shape and based on the detected, preferably real-time, shape, surface and/or orientation detection, identifies possible deviations of the e.g. the current bending of the real implant from the reference bending state or ideal bending state defined by said implantation plan. Thus, this embodiment allows to detect or identify whether certain aspects of the implant shaping process (as part of the implantation plan) are going well, i.e. according to the implantation plan, or not. One of these aspects is for example the "bending parameter", i.e. whether the shape of the real implant during the bending process of the implantation is following the ideal bending process as defined in the implantation plan. Other aspects or parameters of the implanting process, e.g. whether the duration of the bending process is according to the implanting process can be controlled or checked as well with this method and system embodiment.

In other words, this embodiment may use any of the known technologies, like e.g., infrared tracking, mono / stereo camera tracking, time of flight cameras, etc., to identify the implant. Either direct (shape detection) or by additional detecting an attached marker part, e.g. to fix a rotation axis, which can hardly be detected purely by image analysis, looking at a mostly cylindrical object.

According to an embodiment of the present invention the method further comprises the step of visually indicating said detected deviation, preferably by projecting additional elements like colours and/or arrows onto the sterile projection plate by the projection device.

In case the method/system identifies or detects sad at least one deviation from said plan based on a result of said comparing, a corresponding output or signal or information may be accordingly provided to the user, preferably by the projection device. For example, in case a bending deviation is detected, the projection device may project visually additional information onto the sterile plate to transfer this information about the detected bending deviation to the user. This may be done by e.g. signals/information to bend the real implant rather into another direction by using e.g. arrows or by indicating with the projection how the current bending state of the real implant should be adapted to be more or fully in accordance with the implantation plan. Several different ways of projecting such information onto the sterile plate to be recognized by the user can be used.

According to an embodiment of the present invention, the method further comprises the step of providing at least one of an update, a measurement, a quality control, or triggering a replanning of the plan for implanting the planned patient specific implant model.

Detection of an e.g. intermediate bending state of the rod would allow to overlay the current state to the template (= target state), e.g. as a progress indicator or to recognize wrong bending early enough. This could be supported by automated measurements like e.g., image processing. Deviations from the plan could be detected and indicated visually, also by additional projected elements (colours, arrows, etc.). This provision of an update, a measurement, a quality control, or triggering a replanning of the plan for implanting the planned patient specific implant model may be carried out e.g. by the projection device. However, also additional devices may be used to react upon the detected deviation from said plan based on the result of said comparing.

According to an embodiment of the present invention, the method comprises the step of projecting a workflow onto the sterile projection plate by the projection device by projecting intermediate states of the planned patient specific implant model thereby updating the projection of the planned patient specific implant model according to a planned bending progress.

As was mentioned hereinbefore, the method/system is configured to detect the shape, and/or the surface and/or the orientation of the implant and is configured to provide - based on the detected shape, surface and/or orientation - e.g. updates, measurements, quality control, indicator for deviation, and/or replanning information to the user. In this way, the method/system is advantageously configured to adapt the projection to the progress of e.g. a bending process, in which the real implant is bent by the user. Thus, the method/system is configured to provide an intermediate update of the projected implant model according to bending progress, which also provides an adaptation of the projection based on the progress of the ongoing bending process.

According to an embodiment of the present invention, the method further comprises the step of projecting scaling information of implant parameters onto the sterile projection plate.

Note that since the template has to be real-size to work, this embodiment relates to displaying said scaling information like a ruler, numbers etc.. Generally, this embodiment provides two advantages if one displays a ruler. First, a sanity check of the scaling process I provided. A ruler could be "checked" by common sense / physical ruler for correctness. Second, the use of a projected ruler as measurement device for other implants (e.g. screw lengths) to quickly distinguish or check other implants for their dimensions in the sterile field.

According to an embodiment of the present invention, the sterile projection plate is configured to reflect the planned patient specific implant model projected onto the sterile projection plate, and the sterile projection plate is not self-luminous.

In this embodiment, the sterile projection plate is not by itself emitting any light signals, but is just reflecting the light that is illuminated from the projection device onto the sterile plate. Various different materials and shapes may be used for the sterile projection plate. In a particular embodiment, the sterile projection plate is made out of white plastic material and has a rectangular shape. If desired, the sterile projection plate may have grasping recessions in the surface and the left and right end such that the user may easily grasp and move the plate.

According to an embodiment of the present invention, the sterile projection plate is made of a plastic material or aluminium, and preferably the sterile projection plate is of a white colour.

According to an embodiment of the present invention, the sterile projection plate comprises at least one, preferably at least two recessed grips for being grasped by a user of the sterile projection plate.

According to an embodiment of the present invention, the method further comprises the steps
detecting a movement of the sterile projection plate, and
adapting the projecting of the planned patient specific implant model onto the sterile projection plate thereby compensating for the detected movement of the sterile projection plate.

With this embodiment potential real 1D, 2D, and/or 3D movements of the sterile plate, i.e., the sterile projection plate, in the sterile field of the operating can be detected. Based on the detection result, i.e. by knowing which movement the sterile plate has made, the system/method of the present invention can calculate how the projection must be adapted to compensate for such movement. In particular, it can be achieved that the projection of the implant on the sterile projection plate does not change in shape and/or orientation, although the plate is moving. This compensation of sterile plate movement by way of calculation facilitates a real time adaptation of the projection of the implant such that the user can concentrate on using the projection for his implantation task.

In a particular embodiment, the result of the movement detection is used for calculating amended projection parameters for the projection device. This embodiment further entails using said calculated amended projection parameters for adapting the projection of the planned patient specific implant model with the projection device onto the sterile projection plate.

According to an embodiment of the present invention, the method further comprises the step
calculating, based on the detected movement, adapted projection parameters for the projection device,
wherein the adapted projection parameters, when applied by the projection device, compensate for the detected movement of the sterile projection plate.

As is clear to the skilled reader, this embodiment can of course be combined with the embodiment mentioned hereinbefore detailing the generation or calculation of the projection parameters. With the sterile plate movement compensation, the projection will be even more robust and independent from otherwise disturbing changes of the position and/or orientation of the sterile plate relative to the detection device and/or relative to the projection device. As will be appreciated from the present disclosure, a "live calibration" is carried out, i.e., that a moving plate can be compensated for by the system by detecting said movement and by accordingly adapting the projection.

According to another aspect of the present invention, a system for generating a projection of a planned patient specific implant model on a sterile projection plate in a sterile field is presented. The system comprises
a detection device configured for detecting a sterile projection plate for being located in the sterile filed,
a planned patient specific implant model stored on the system or accessible by the system, and
a projection device configured for projecting the planned patient specific implant model onto the sterile projection plate.

According to an embodiment of the present invention the detection device is configured for determining, upon detecting the sterile projection plate located in the sterile field with the detection device, a relative position and a relative orientation of the sterile projection plate to the detection device. Preferably the determined relative position and the determined relative orientation of the sterile projection plate relative to the detection device facilitate a correct scaling and an undistorted projection of the planned patient specific implant model onto the sterile projection plate.

According to an embodiment of the present invention, the system is configured for calculating, based on a result of the detection of the sterile projection plate, projection parameters for the projection device, and using said calculated projection parameters when projecting the planned patient specific implant model with the projection device onto the sterile projection plate. Preferably using said calculated projection parameters ensures a correct scaling and an undistorted projection of the planned patient specific implant model onto the sterile projection plate.

According to an embodiment of the present invention the detection device is configured for generating at least one image of the sterile projection plate, wherein identification information about the sterile projection plate is stored in the detection device and/or in the projection device and/or in a code, like a QR code, on the plate, and wherein the detection device is further configured for identifying the sterile projection plate based on a comparison between the stored identification information and content of the generated at least one image. Preferably the stored identification information is size information about a size of the sterile projection plate, and wherein the system is configured for calculating, based on the generated at least one image and the size information, projection parameters for the projection device for projecting the planned patient specific implant model onto the sterile projection plate.

According to an embodiment of the present invention the detection device is further configured for using one or more further identification parameters of the sterile projection plate during the detection of the sterile plate for increasing a robustness of the detection.

According to an embodiment of the present invention the detection device is a video camera, and/or wherein the projection device is a laser projection device.

According to an embodiment of the present invention the detection device is calibrated to the projection device with respect to their spatial relative position and/or and their relative angular orientation.

According to an embodiment of the present invention the projection device is configured for receiving tracking data indicative of a position and/or orientation of a real implant or of a surgical instrument, and projecting the planned patient specific implant model in different orientations onto the sterile projection plate with the projection device based on the received tracking data. The tracking data preferably are video-tracking data and/or marker-based tracking data.

According to an embodiment of the present invention the system is configured for detecting, by the detection device, a shape, a surface and/or an orientation of a real implant, preferably using an object detection algorithm, comparing the detected shape, surface and/or orientation of the real implant with a plan for implanting the planned patient specific implant model, and detecting at least one deviation from said plan based on a result of said comparing. Preferably the system is configured for visually indicating said detected deviation, further preferably by projecting additional elements like colours and/or arrows onto the sterile projection plate by the projection device. Further preferably, the system is configured for providing at least one of an update, a measurement, a quality control, or triggering a replanning of the plan for implanting the planned patient specific implant model.

According to an embodiment of the present invention the projection device is configured for projecting a workflow onto the sterile projection plate by projecting intermediate states of the planned patient specific implant model thereby updating the projection of the planned patient specific implant model according to a planned bending progress.

According to an embodiment of the present invention the projection device is configured for projecting scaling information of implant parameters onto the sterile projection plate.

According to an embodiment of the present invention the sterile projection plate is configured to reflect the planned patient specific implant model projected onto the sterile projection plate, and wherein the sterile projection plate is not self-luminous. Preferably the sterile projection plate is made of a plastic material or aluminium. Preferably, the sterile projection plate is of a white colour.

According to an embodiment of the present invention the sterile projection plate comprises at least one, preferably at least two recessed grips for being grasped by a user of the sterile projection plate.

According to an embodiment of the present invention the system is configured for detecting a movement of the sterile projection plate, adapting the projecting of the planned patient specific implant model onto the sterile projection plate thereby compensating for the detected movement of the sterile projection plate.

Preferably the system is configured for calculating, based on the detected movement, adapted projection parameters for the projection device, and wherein the adapted projection parameters, when applied by the projection device, compensate for the detected movement of the sterile projection plate.

According to an embodiment of the present invention, the system further comprises the sterile projection plate.

According to an embodiment of the present invention, the system is configured to carry out the method detailed hereinbefore.

For example, the invention does not involve or in particular comprise or encompass an invasive step which would represent a substantial physical interference with the body requiring professional medical expertise to be carried out and entailing a substantial health risk even when carried out with the required professional care and expertise. For example, the invention does not comprise a step of positioning a medical implant in order to fasten it to an anatomical structure or a step of fastening the medical implant to the anatomical structure or a step of preparing the anatomical structure for having the medical implant fastened to it. More particularly, the invention does not involve or in particular comprise or encompass any surgical or therapeutic activity. For this reason alone, no surgical or therapeutic activity and in particular no surgical or therapeutic step is necessitated or implied by carrying out the invention.

### DEFINITIONS

In this section, definitions for specific terminology used in this disclosure are offered which also form part of the present disclosure.

### Acquiring data/an image

The expression "acquiring data" and/or "acquiring an image" (which will be used herein synonymously) for example encompasses (within the framework of a computer implemented method) the scenario in which the data/image data are determined by the computer implemented method or program. Determining data for example encompasses measuring physical quantities and transforming the measured values into data, for example digital data, and/or computing (and e.g. outputting) the data by means of a computer and for example within the framework of the method in accordance with the invention. The meaning of "acquiring data"/" acquiring an image" also for example encompasses the scenario in which the data are received or retrieved by (e.g. input to) the computer implemented method or program, for example from another program, a previous method step or a data storage medium, for example for further processing by the computer implemented method or program. Generation of the data to be acquired may but need not be part of the method in accordance with the invention. The expression "acquiring data" can therefore also for example mean waiting to receive data and/or receiving the data. The received data can for example be inputted via an interface. The expression "acquiring data" can also mean that the computer implemented method or program performs steps in order to (actively) receive or retrieve the data from a data source, for instance a data storage medium (such as for example a ROM, RAM, database, hard drive, etc.), or via the interface (for instance, from another computer or a network). The data acquired by the disclosed method or device, respectively, may be acquired from a database located in a data storage device which is operably to a computer for data transfer between the database and the computer, for example from the database to the computer. The computer acquires the data for use as an input for steps of determining data. The determined data can be output again to the same or another database to be stored for later use. The database or database used for implementing the disclosed method can be located on network data storage device or a network server (for example, a cloud data storage device or a cloud server) or a local data storage device (such as a mass storage device operably connected to at least one computer executing the disclosed method). The data can be made "ready for use" by performing an additional step before the acquiring step. In accordance with this additional step, the data are generated in order to be acquired. The data are for example detected or captured (for example by an analytical device). Alternatively or additionally, the data are inputted in accordance with the additional step, for instance via interfaces. The data generated can for example be inputted (for instance into the computer). In accordance with the additional step (which precedes the acquiring step), the data can also be provided by performing the additional step of storing the data in a data storage medium (such as for example a ROM, RAM, CD and/or hard drive), such that they are ready for use within the framework of the method or program in accordance with the invention. The step of "acquiring data" can therefore also involve commanding a device to obtain and/or provide the data to be acquired. In particular, the acquiring step does not involve an invasive step which would represent a substantial physical interference with the body, requiring professional medical expertise to be carried out and entailing a substantial health risk even when carried out with the required professional care and expertise. In particular, the step of acquiring data, for example determining data, does not involve a surgical step and in particular does not involve a step of treating a human or animal body using surgery or therapy. In order to distinguish the different data used by the present method, the data are denoted (i.e. referred to) as "XY data" and the like and are defined in terms of the information which they describe, which is then preferably referred to as "XY information" and the like.

### BRIEF DESCRIPTION OF THE DRAWINGS

In the following, the invention is described with reference to the appended figures which give background explanations and represent specific embodiments of the invention. The scope of the invention is however not limited to the specific features disclosed in the context of the figures, wherein
- Fig. 1: illustrates a system for generating a projection of a planned patient specific implant model on a sterile projection plate in a sterile field according to an exemplary embodiment; and
- Fig. 2: shows a flow diagram of a method of generating a projection of a planned patient specific implant model on a sterile projection plate in a sterile field according to an exemplary embodiment.
- Fig.3: illustrates a system for generating a projection of a planned patient specific implant model on a sterile projection plate in a sterile field according to another exemplary embodiment
- Fig. 4: illustrates a sterile plate which can be used together with the present invention.

### DESCRIPTION OF EMBODIMENTS

In the following, technical features used in the context of the present invention and associated technical effects are described.

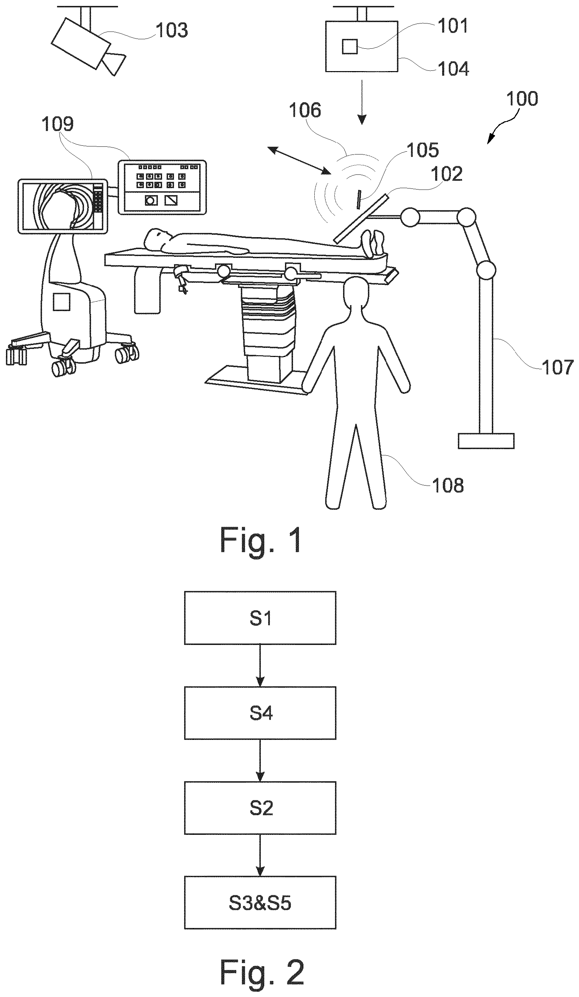

Figure 1 schematically shows a system 100 for generating a projection 105 of a planned patient specific implant model 101 on a sterile projection plate 102 in a sterile field of an operating room according to an exemplary embodiment. The plate is fixated at device 107 standing beside a user 108. Figure 1 also shows displays 109, which can be used by the user 108 while the presented invention is applied. The model 101 is stored in this embodiment in the projection device 104 in the form of a digital file. The system 100 is configured for generating a projection 105 of a planned patient specific implant model 101 on the sterile projection plate 102. The projection is indicated by electromagnetic waves 106. The detection device 103 is configured for detecting a sterile projection plate 102 for providing the planned patient specific implant model to a device 104, and the projection device 104 is configured for projecting the planned patient specific implant model 101 onto the sterile projection plate. In an embodiment of the present invention, the system 101 is configured for detecting a movement of the sterile projection plate 102, and is configured for adapting the projection 105 of the planned patient specific implant model 101 onto the sterile projection plate 102 thereby compensating for the detected movement of the sterile projection plate 102. In another preferred embodiment of the present invention, the system 100 is configured for calculating, based on the detected movement, adapted projection parameters for the projection device 104, wherein the adapted projection parameters, when applied by the projection device 104, compensate for the detected movement of the sterile projection plate 102. In the system 100 detecting the projection plate with a detection device 103 is carried out with a video camera. Thus, the method/system 100 gathers knowledge about the relative position and orientation of the sterile projection plate 102 to the detection device 103, which may be session specific. If desired, this information can be used for correct scaling and undistorted display. In other words, due to the detecting of the projection plate 102 with the camera 103, the projection device 104 can project the planned model 101 in the correct size.

A calibration of the camera 103 to the laser projector 104 is carried out frequently. Thus, the method/system 100 gathers information about the relative position and orientation of the projection device 103 to the detection device 104 and thus to the sterile projection plate 102. Moreover, in the method presented herein, the step of providing a planned patient specific implant model is caried out, e.g. a rod, a patient specific model. The user and/or the system 100 may compare the real implant (not shown) to the projected model 105, i.e. the template 105, which allows the method and system 100 to carry out a progress and/or success control of the desired bending of the real implant. The method and system 100 may thus use pre-defined information about the desired or ideal bending process and compare this information with the real bending detected/surveyed by the detection device. In other words, if in an embodiment a video camera 103 is used as the detection device, the system 100 may be configured to detect in video images of the video camera the current shape, form and/or surface of the real implant and this may be compared with said pre-defined information. This pre-defined information may be stored in the system 100 but may also be stored remotely on e.g. a server to which the 100 can communicate. If desired, scaling information may be projected or displayed to the user by the system 100, e.g. the projection device 104. In other words, augmented information of relevant implant parameters may be provided by the method/system for the user.

In particular embodiments, the method and system 100 is configured to detect the shape of the real implant (not shown) and/or the surface of the implant and/or the orientation of the implant and is configured to provide - based on the detected shape, and/or surface and/or orientation - updates, measurements, quality control, indicator for deviation, and/or replanning information to user, preferably by the projection device 104. In this way, the method and system 100 is advantageously configured to adapt to the progress of e.g. a bending process, in which the real implant is bent by the user. Moreover, the method/system is configured to provide an intermediate update of the implant model according to bending progress, which also provides an adaptation of the projection based on the progress of the ongoing bending process. In a particular, embodiment, method and system 100 tracks the real implant by e.g. video camera 103 or with marker-based technique to show correct position and orientation. This allows displaying of multiple or arbitrary views on the implant. Note that the projection device 104 and detection device 103 can be separate devices, but can also be combined in a fixed configuration, i.e., in one housing. The combination in one housing may be precalibrated. Further, an additional tracking device (not shown), which is separate from the detection device 103, may be used allowing for tracking of real implants with any tracking and/or marker technology.

Figure 2 shows a flow diagram of a method of generating a projection of a planned patient specific implant model 101 on a sterile projection plate 102 in a sterile field. The method comprises the steps of detecting the sterile projection plate 102 located in the sterile field with a detection device 103 (step S1) and calculating, based on a result of the detection of the sterile projection plate carried out in step S1, projection parameters for the projection device (step S4). Further, the planned patient specific implant model is provided to a projection device 104 (step S2). Further the step of projecting the planned patient specific implant model 101 with the projection device 104 onto the sterile projection plate (step S3) is comprised, wherein said calculated projection parameters in step S3 when projecting the planned patient specific implant model with the projection device onto the sterile projection plate (step S5). The method shown in Figure 1 can be carried out by a system as shown in Figure 1, but also other systems may be used.

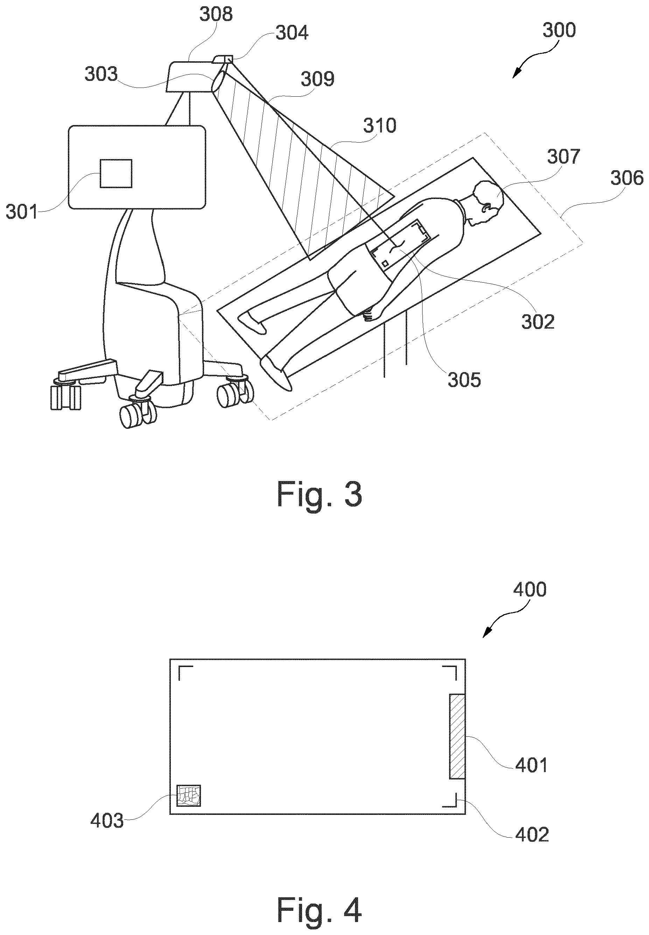

Figure 3 schematically shows a system 300 for generating a projection 305 of a planned patient specific implant model 301 on a sterile projection plate 302 lying on a patient 307 in a sterile field 306 of an operating room according to an exemplary embodiment. In this embodiment the planned patient specific implant model 301 is stored in the device combining the camera detection device 303 and the laser light projection device 304 within a combined housing 308. The laser light projection device 304 projects laser/light projection 309, while the camera detection device 303 has a camera filed of view 310.

Figure 4 illustrates a sterile plate 400 which can be used together with the present invention. The sterile plate 400 provides a grasping recession 401 for grasping the recession, i.e., for grasping the sterile plate, by a user. Additionally, further identification parameters of the sterile projection plate 400 are provided on the surface of the sterile plate 400 that can be detected by the detection device, e.g. 303 in order increase robustness of the detection process, i.e. the detection result. The plate 400 also provides a QR code 403, with which size information and shape information about the plate 400 can be stored and retrieved.

## Claims

1. A system (100, 300) for generating a projection (105, 305) of a planned patient specific implant model (101, 301) on a sterile projection plate (102, 302) in a sterile field, the system comprising:
a detection device (103, 303) configured for detecting a sterile projection plate (102, 302) for being located in the sterile filed (306),
a planned patient specific implant model (101, 301) stored on the system or received by the system, and
a projection device (104, 304) configured for projecting the planned patient specific implant model onto the sterile projection plate.

2. The system according to claim 1,
wherein the detection device is configured for determining, upon detecting the sterile projection plate (102) located in the sterile field with the detection device (103), a relative position and a relative orientation of the sterile projection (102) plate to the detection device (103),
wherein preferably the determined relative position and the determined relative orientation of the sterile projection plate (101) relative to the detection device (103) facilitate a correct scaling and an undistorted projection of the planned patient specific implant model onto the sterile projection plate.

3. The system according to any of the preceding claims, being configured for
calculating, based on a result of the detection of the sterile projection plate, projection parameters for the projection device, and
using said calculated projection parameters when projecting the planned patient specific implant model with the projection device onto the sterile projection plate,
wherein preferably using said calculated projection parameters ensures a correct scaling and an undistorted projection of the planned patient specific implant model onto the sterile projection plate.

4. The system according to any of the preceding claims,
wherein the detection device (103, 303) is configured for generating at least one image of the sterile projection plate,
wherein identification information about the sterile projection plate is stored in the detection device and/or in the projection device and/or in a code, like a QR code, on the plate, and
wherein the detection device (103, 303) is further configured for identifying the sterile projection plate based on a comparison between the stored identification information and content of the generated at least one image,
wherein preferably the stored identification information is size information about a size of the sterile projection plate, and
wherein the system is configured for calculating, based on the generated at least one image and the size information, projection parameters for the projection device for projecting the planned patient specific implant model onto the sterile projection plate.

5. The system according to any of the preceding claims,
wherein the detection device is further configured for using one or more further identification parameters of the sterile projection plate during the detection of the sterile plate for increasing a robustness of the detection.

6. The system according to any of the preceding claims,
wherein the detection device is a video camera, and/or
wherein the projection device is a laser projection device.

7. The system according to any of the preceding claims, wherein the detection device is calibrated to the projection device with respect to their spatial relative position and/or and their relative angular orientation.

8. The system according to any of the preceding claims, wherein the projection device (104, 304) is configured for
receiving tracking data indicative of a position and/or orientation of a real implant or of a surgical instrument, and
projecting the planned patient specific implant model in different orientations onto the sterile projection plate with the projection device based on the received tracking data,
wherein the tracking data preferably are video-tracking data and/or marker-based tracking data.

9. The system according to any of the preceding claims, wherein system is configured for
detecting, by the detection device, a shape, a surface and/or an orientation of a real implant, preferably using an object detection algorithm,
comparing the detected shape, surface and/or orientation of the real implant with a plan for implanting the planned patient specific implant model, and
detecting at least one deviation from said plan based on a result of said comparing,
preferably visually indicating said detected deviation, further preferably by projecting additional elements like colours and/or arrows onto the sterile projection plate by the projection device,
further preferably providing at least one of an update, a measurement, a quality control, or triggering a replanning of the plan for implanting the planned patient specific implant model.

10. The system according to any of the preceding claims, wherein the projection device is configured for projecting a workflow onto the sterile projection plate by projecting intermediate states of the planned patient specific implant model thereby updating the projection of the planned patient specific implant model according to a planned bending progress.

11. The system according to any of the preceding claims, wherein the projection device is configured for projecting scaling information of implant parameters onto the sterile projection plate.

12. The system according to any of the preceding claims,
wherein the sterile projection plate is configured to reflect the planned patient specific implant model projected onto the sterile projection plate, and
wherein the sterile projection plate is not self-luminous,
wherein preferably the sterile projection plate is made of a plastic material or aluminium, and preferably the sterile projection plate is of a white colour.

13. The system according to any of the preceding claims,
wherein the sterile projection plate comprises at least one, preferably at least two recessed grips for being grasped by a user of the sterile projection plate.

14. The system according to any of the preceding claims, being configured for
detecting a movement of the sterile projection plate,
adapting the projecting of the planned patient specific implant model onto the sterile projection plate thereby compensating for the detected movement of the sterile projection plate,
preferably calculating, based on the detected movement, adapted projection parameters for the projection device, and
wherein the adapted projection parameters, when applied by the projection device, compensate for the detected movement of the sterile projection plate.

15. A method of generating a projection of a planned patient specific implant model (101) onto a sterile projection plate (102) in a sterile field, the method comprising the steps
detecting a sterile projection plate (102) located in the sterile field with a detection device (103) (step S1),
providing the planned patient specific implant model to a projection device (104) (step S2), and
projecting the planned patient specific implant model (101) with the projection device (104) onto the sterile projection plate (step S3).
